(19) Europäisches Patentamt
European Patent Office
Office européen des brevets

(11) Publication number: 0 117 459
B1

(12) # EUROPEAN PATENT SPECIFICATION

(45) Date of publication of patent specification: 06.09.89

(51) Int. Cl.⁴: **C 07 C 37/66**, C 07 C 39/235

(21) Application number: 84101177.8

(22) Date of filing: 06.02.84

(54) **Process for preparing anhydrous salts of dihydroxy aromatic compounds.**

(30) Priority: 28.02.83 US 470856

(43) Date of publication of application:
05.09.84 Bulletin 84/36

(45) Publication of the grant of the patent:
06.09.89 Bulletin 89/36

(84) Designated Contracting States:
DE FR GB IT NL

(56) References cited:
US-A-3 974 084

(73) Proprietor: GENERAL ELECTRIC COMPANY
1 River Road
Schenectady New York 12305 (US)

(72) Inventor: Mendiratta, Ashok Kumar
1317 Viewmont Drive
Schenectady New York 12309 (US)
Inventor: Sikdar, Subbas Kumar
46 Appletree Lane
Clifton Park New York 12065 (US)

(74) Representative: Catherine, Alain
General Electric France Service de Propriété
Industrielle 18 Rue Horace Vernet B.P. 76
F-92134 Issy-les-Moulineaux Cedex (FR)

EP 0 117 459 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the preparation of di-(alkali metal) salts of dihydroxy aromatic compounds and to a method of recovery thereof. In its broadest sense, the invention is directed to a process for recovering such salts in anhydrous form which comprises:

(A) contacting at a temperature below 75°C solid dihydroxy aromatic compound having the formula HO—Z—OH, wherein Z has one of the following formulas:

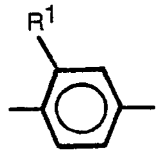

(I)

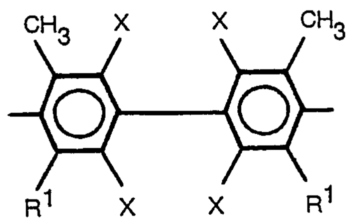

(II)

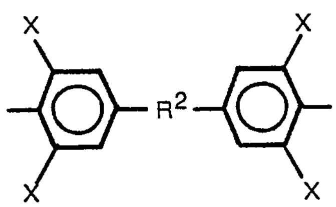

(III)

in these formulas, each $R^1$ is independently hydrogen or methyl, $R^2$ is a straight-chain or branched alkylene radical containing 1—5 carbon atoms and is most often the isopropylidene radical, and each X is independently hydrogen or halogen with an aqueous solution of an alkali metal hydroxide for a period of time sufficient to convert said compound to its di-(alkali metal) salt;

(B) separating said solid salt or its hydrate from the aqueous system;

(C) mixing said solid salt or hydrate with an organic liquid having a boiling point higher than that of water or which forms an azeotrope with water to create a system adapted for removal of water, including water of hydration, therefrom by evaporation; and

(D) evaporating said water from said system.

The preparation of di-(alkali metal) salts of dihydroxy aromatic compounds typically and conveniently takes place in an aqueous system. Many commercially important reactions of said salts, however, such as their reaction with nitrophthalimides to form aromatic ether imides, are best carried out under anhydrous conditions to maximize yields. It is necessary, therefore, to isolate the salt in anhydrous form.

Various methods for removal of water from such salts have been disclosed. For example, US—A—4,202,993 describes a flash evaporation method, while US—A—4,257,953 discloses azeotropic distillation of the water using a hydrocarbon solvent such as toluene. While these methods are effective, they suffer from certain disadvantages. For example, the presence of alkali metal hydroxide may be detrimental in subsequent reactions, such as the reaction with nitrophthalimide. Since no measures are taken in these methods to remove alkali metal hydroxide, it is necessary to carefully monitor and maintain at stoichiometric the proportion thereof used for converting the dihydroxy aromatic compound to its di-(alkali metal) salt. Also, a large amount of toluene is required to insure the azeotropic removal of the relatively large quantity of water (often about 2—3 parts by weight per part of dihydroxy aromatic compound) used in the reaction, and the di-(alkali metal) salt is susceptible to caking during the removal of water.

A principal object of the present invention, therefore, is to provide an improved process for the preparation of di-(alkali metal) salts of dihydroxy aromatic compounds.

A further object is to provide an improved method for the removal of water from such salts.

A further object is to provide a method for recovery of such salts in anhydrous form as free-flowing solids, adapted for easy handling during further reactions.

Still another object is to provide a method for the preparation of such salts which is flexible and involves a minimum of operations and reaction monitoring steps.

A still further object is to prepare such salts by a method involving a minimum of energy expenditure for heating and the like.

Other objects will in part be obvious and will in part appear hereinafter.

The dihydroxy aromatic compounds which may be converted to alkali metal salts according to the process of this invention generally have the formula HO—Z—OH, wherein Z has one of the following formulas:

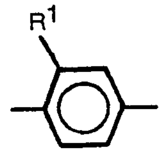

(I)

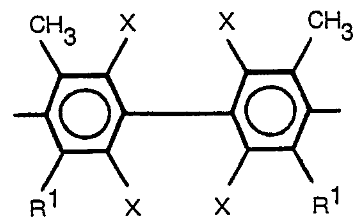

(II)

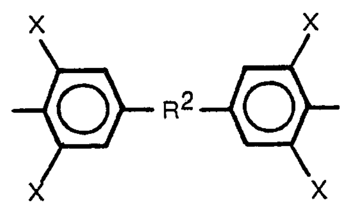

(III)

In these formulas, each $R^1$ is independently hydrogen or methyl, $R^2$ is a straight-chain or branched alkylene radical containing 1—5 carbon atoms and is most often the isopropylidene radical, and each X is independently hydrogen or halogen (usually chlorine or bromine). The compounds in which Z has formula III are bisphenols. Since the invention is particularly useful for the preparation of bisphenol salts, frequent reference to bisphenols will be made hereinafter. However, the invention can also be used to prepare salts of compounds in which Z has formula I or II, or of mixtures of compounds in which Z has any or all of these formulas. The preferred bisphenol is bisphenol A, i.e., 2,2-bis-(4-hydroxyphenyl)propane, which has formula III wherein $R^2$ is isopropylidene and each X is hydrogen.

The di-(alkali metal) salts which are formed by the process of this invention are those of the metals of Group IA of the periodic table; namely, lithium, sodium, potassium, rubidium and cesium. For reasons of economy and availability, the sodium and potassium salts, especially the former, are preferred. Therefore, reference hereinafter will frequently be made to sodium as the alkali metal used. It should be understood, however, that other alkali metals can be substituted for sodium.

In step A of the process of this invention, the solid bisphenol is contacted with an aqueous sodium hydroxide solution. This solution typically comprises 15—50% by weight, preferably 15—30%, sodium hydroxide. The weight ratio of water to bisphenol during this step is usually from 2:1 to 3:1.

Contact during step A is typically effected at a temperature below about 75° and especially below about 45°C, preferably within the range of 10—50° and most desirably 25—40°C. At temperatures within this range, the bisphenol is substantially insoluble in the aqueous system. Nevertheless, it undergoes a heterogeneous reaction with the sodium hydroxide to yield the desired disodium salt, which in this case separates as the solid hydrate which is also substantially insoluble at these temperatures. Nevertheless, the process of this invention is not limited to salts which form hydrates; it may also be used to prepare other salts which are substantially insoluble under the conditions described.

It is ordinarily desirable to achieve substantially complete conversion of the bisphenol to its sodium salt. Therefore, at least a stoichiometric amount of sodium hydroxide (i.e., at least one equivalent per equivalent of bisphenol) should be used. In order to insure completeness of the reaction, it is frequently advantageous to use up to about 20% excess sodium hydroxide, most often up to about 10%. One of the advantages of this invention is that excess sodium hydroxide may be used without adversely affecting the product.

The time required for completion of step A is normally about 2—4 hours. The reaction will, of course, be faster at higher temperatures within the preferred range, but hydrate recovery may be lower because of increased salt or hydrate solubility in water at higher temperatures. Optimum conditions are frequently attained at 25—40°C and a reaction time of about 3—4 hours.

Step B comprises separation of the solid salt or hydrate from the aqueous system. Separation is typically accomplished by known processes such as filtration or centrifugation.

In step C, the solid salt or hydrate obtained in step B is mixed with an organic liquid to create a system

adapted for removal of water therefrom by evaporation. A wide variety of organic liquids can be used; in general, they comprise all liquids which have a boiling point higher than that of water (i.e., higher than 100°C) or which form azeotropes with water. These conditions are applicable because a liquid which has a lower boiling point than water and does not form an azeotrope therewith will itself be removed by evaporation before the water is removed.

Typical organic liquids which may be used in step C include benzene, toluene, xylene, chlorobenzene, o-dichlorobenzene, heptane, octane, nonane, decane, petroleum naphthas with a higher boiling point than that of water, chloroform, carbon tetrachloride, ethylene glycol, ethylene glycol monoethyl ether, diethylene glycol, diethylene glycol dimethyl ether, diethylene glycol dibutyl ether, 2-butanol, dioxane and methyl isobutyl ketone. While the degree of solubility of water in the organic liquid is not critical, it is preferred to use liquids in which water is substantially insoluble and which boil above 105°C and preferably below about 200°C, and especially those in which the bisphenol sodium salt is substantially insoluble. The aromatic hydrocarbons and chlorinated hydrocarbons (e.g., benzene, toluene, xylene, chlorobenzene, o-dichlorobenzene) are especially suitable; of these, toluene and o-chlorobenzene are most desirable because of their effectiveness, availability and relatively low price.

The weight ratio of organic liquid to salt or hydrate used in step C depends to some extent on the creation or non-creation of an azeotropic mixture, the proportions of the liquids in that mixture, and the creation of a free-flowing slurry of the salt hydrate. Generally, about 1—3 parts by weight of organic liquid per part of salt or hydrate is sufficient. The preferred ratio is between about 1.5:1 and about 2:1.

Drying and/or dehydration of the solid salt or hydrate is effected in step D by evaporation. The evaporative process used may be flash evaporation, distillation or any other suitable type. Often, a portion of the organic liquid is simultaneously removed by evaporation, especially when it forms an azeotrope with water, but evaporation of the organic liquid is not an essential step of the process. When a water-immiscible liquid such as toluene is used, it is frequently convenient to introduce said liquid at an elevated temperature, typically about 100°C, continuously or intermittently until the condensate no longer contains a substantial amount of water. Typically, drying is complete when said condensate contains less than about 100 ppm and preferably less than about 50 ppm of water.

Following step D, the dry anhydrous sodium salt may be separated from the organic liquid. The method of separation will vary according to whether the di-(alkali metal) salt is soluble or insoluble in the organic liquid. If it is soluble, the liquid may typically be removed by evaporation at atmospheric or reduced pressure. In the preferred embodiment which employs a liquid in which the anhydrous disodium salt is substantially insoluble, separation is relatively simple and may be effected by filtration, centrifugation or the like. Remaining traces of organic liquid in the salt may be removed by vacuum drying or a similar operation. It is, however, often most convenient to employ the salt in slurry form. An illustration is its reaction with a nitrophthalimide. For such uses, separation of the salt from the organic liquid is unnecessary.

The process of this invention has many advantages over previously known processes involving such expedients as azeotropic removal of all the water in which the sodium salt was dissolved. In the first place, as previously mentioned, it is possible to employ an excess of sodium hydroxide in the formation of the sodium salt of the bisphenol. This fact, combined with the rapidity of water removal, substantially decreases the time required for salt preparation. In the second place, according to the process of this invention as much as 70% by weight of the total water in the system is removed relatively simply in step B, with a relatively small amount remaining for removal in step D; therefore, energy usage for vaporization in step D is minimized. In the third place, the anhydrous bisphenol disodium salt is obtained as a free-flowing solid with little or no tendency to agglomerate or cake. In the fourth place, the process can be adapted for continuous operation.

Reference is now made to the drawing which depicts a typical reaction system for the practice of the process of this invention. Reactor 1 is a tank-type reactor fitted with stirring means 2 and also with temperatrure control means (not shown), and typically may also contain baffles (not shown) to insure thorough agitation of the contents. Aqueous sodium hydroxide, bisphenol and water are introduced into reactor 1 at 3, 4 and 5 respectively; typically, an inert atmosphere is maintained therein by the addition of an inert gas such as nitrogen at 6. The mixture in reactor 1 is agitated for a period of time sufficient to convert substantially all the bisphenol to its disodium salt; the aqueous slurry of the salt hydrate is pumped through line 7 into separation means 8, typically a filter or centrifuge. The mother liquor is recycled through line 9 to reactor 1. If desired, water may be added via line 10 to wash the hydrate crystals. If this is done, such water is normally part of the feed water introduced at 5 to avoid dilution of the sodium hydroxide solution in reactor 1.

The solid hydrate from separation means 8 passes through line 11 into stirred separation vessel 12, which is also typically maintained in an inert atmosphere by the introduction of nitrogen or a similar inert gas at 13. The organic liquid, typically toluene or o-dichlorobenzene, is introduced at 14 and the mixture is stirred and heated by temperature control means (not shown). Water and organic liquid are removed by evaporation at 15 and separated by liquid-liquid separation means 16, the organic liquid being returned via line 17 to separation vessel 12. When water removal is substantially complete the slurry of anhydrous bisphenol disodium salt in the organic liquid is drained through line 18. It may be used in slurry form or separated by conventional means from the organic liquid, which may then be recycled.

The invention is illustrated by the following examples. All parts are by weight.

Example 1

A mixture of 700 parts (3.07 moles) of solid bisphenol A, 590 parts of 50% aqueous sodium hydroxide solution (7.38 moles of sodium hydroxide or a 20% excess) and 1400 parts of water was prepared in a reactor equipped with vertical baffles and a pitched blade turbine impeller. The reaction between the sodium hydroxide and bisphenol A was conducted for 2.5 hours at 35°C. The contents of the reactor were centrifuged and 950 parts of wet solids were recovered; analysis of the solids showed that only negligible amounts of bisphenol A, sodium hydroxide and the monosodium salt were present therein. The solid cake was returned to the reactor and 1950 parts of toluene were added. The contents of the reactor were evaporated, with periodic addition of hot toluene to maintain the liquid level. When the liquid removed by evaporation contained less than 50 ppm of water, the toluene-salt slurry was removed. The bisphenol A disodium salt therein was in finely divided, free-flowing form with no lumps or cakes.

Example 2

The procedure of Example 1 was repeated, substituting o-dichlorobenzene on an equal weight basis for toluene. The results were similar, except that water removal was faster and requires less organic liquid owing to the higher boiling point of o-dichlorobenzene.

**Claims**

1. A process for recovering in anhydrous form a hydrate-forming di-(alkali metal) salt of a dihydroxy aromatic compound characterized in that it comprises:

(A) contacting at a temperature below 75°C solid dihydroxy aromatic compound having the formula HO—Z—OH, wherein Z has one of the following formulas:

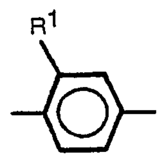

(I)

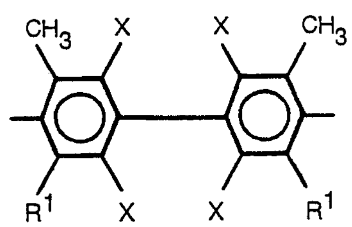

(II)

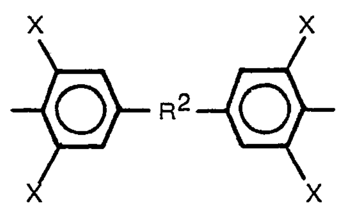

(III)

in these formulas, each $R^1$ is independently hydrogen or methyl, $R^2$ is a straight-chain or branched alkylene radical containing 1—5 carbon atoms and is most often the isopropylidene radical, and each X is independently hydrogen or halogen with an aqueous solution of an alkali metal hydroxide for a period of time sufficient to convert said compound to its di-(alkali metal) salt;

(B) separating said solid salt or its hydrate from the aqueous system;

(C) mixing said solid salt or hydrate with an organic liquid having a boiling point higher than that of water or which forms an azeotrope with water to create a system adapted for removal of water, including water of hydration, therefrom by evaporation; and

(D) evaporating said water from said system.

2. A process according to claim 1 wherein the alkali metal is sodium.

3. A process according to claim 1 wherein step A is effected in aqueous slurry at a temperature below about 45°C.

4. A process according to claim 3 wherein the organic liquid used in step C is one in which water and the anhydrous disodium salt are substantially insoluble.

5. A process according to claim 4 wherein the organic liquid boils above 105°C.

6. A process according to claim 5 wherein the organic liquid is toluene or o-dichlorobenzene.

7. A process according to claim 6 wherein an excess of sodium hydroxide up to about 20% is used in step A.

8. A process according to claim 7 wherein the organic liquid is toluene.

9. A process according to claim 7 wherein the organic liquid is o-dichlorobenzene.

10. A process according to any of claims 1—9 wherein the dihydroxy aromatic compound is bisphenol A.

**Patentansprüche**

1. Verfahren zum Gewinnen eines hydratbildenden Di(alkali-metall)-salzes einer aromatischen Dihydroxyverbindung in wasserfreier Form, gekennzeichnet durch:

(A) in Berührung bringen einer festen aromatischen Di-hydroxyverbindung der Formel HO—Z—OH, worin Z eine der folgenden Formeln hat

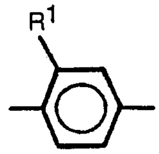

(I)

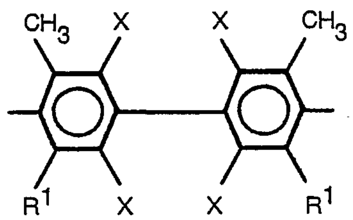

(II)

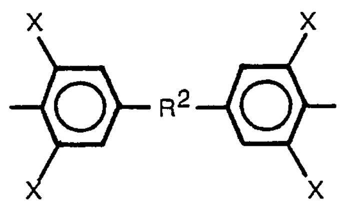

(III)

worin jedes $R^1$ unabhängig Wasserstoff oder Methyl ist, $R^2$ ein geradkettiger oder verzweigter Alkylenrest mit 1 bis 5 Kohlenstoffatomen und am häufigsten der Isopropylidenrest ist und jedes X unabhängig Wasserstoff oder Halogen ist,
mit einer wässrigen Lösung eines Alkalimetallhydroxids bei einer Temperatur unterhalb von 75°C für eine Zeitdauer, die ausreicht, die genannte Verbindung in ihr festes Di(alkalimetall)-salz umzuwandeln;

(B) Abtrennen des genannten festen Salzes oder seines Hydrates vom wässrigen System;

(C) Vermischen des genannten festen Salzes oder seines Hydrates mit einer organischen Flüssigkeit, die einen Siedepunkt hat, der höher als der von Wasser ist oder die ein azeotropes Gemisch mit Wasser bildet, um ein System zu schaffen, das für die Entfernung von Wasser, einschließlich des Hydratationswassers, daraus durch Verdampfen geeignet ist und

(D) Verdampfen des Wassers aus dem genannten System.

2. Verfahren nach Anspruch 1, worin das Alkalimetall Natrium ist.

3. Verfahren nach Anspruch 1, worin Stufe (A) in einer wässrigen Aufschlämmung bei einer Temperatur unterhalb von etwa 45°C ausgeführt wird.

4. Verfahren nach Anspruch 3, worin die in Stufe (C) benutzte organische Flüssigkeit eine ist, in der Wasser und das wasserfreie Dinatriumsalz im wesentlichen unlöslich sind.

5. Verfahren nach Anspruch 4, worin die organische Flüssigkeit oberhalb von 105°C siedet.

6. Verfahren nach Anspruch 5, worin die organische Flüssigkeit Toluol oder o-Dichlorbenzol ist.

7. Verfahren nach Anspruch 6, worin in Stufe (A) ein Überschuß an Natriumhydroxid von bis zu etwa 20% benutzt wird.

8. Verfahren nach Anspruch 7, worin die organische Flüssigkeit Toluol ist.

9. Verfahren nach Anspruch 7, worin die organische Flüssigkeit o-Dichlorbenzol ist.

10. Verfahren nach irgendeinem der Ansprüche 1 bis 9, worin die aromatische Dihydroxyverbindung Bisphenol-A ist.

**Revendications**

1. Procédé de récupération sous forme anhydre d'un die-sel de métal alcalin d'un composé aromatique

dihydroxylé formant un hydrate caractérisé en ce qu'il comprend:

(A) la mise en contact à une température inférieure à 75°C d'un composé aromatique dihydroxylé solide ayant pour formule HO—Z—OH, dans laquelle Z répond à une des formules suivantes:

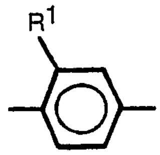

(I)

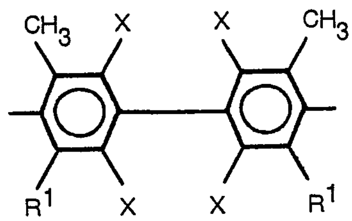

(II)

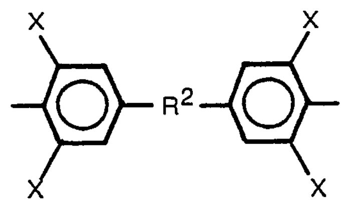

(III)

sachant que dans ces formules, chacun des groupes $R^1$ représente indépendamment l'hydrogène ou un radical méthyle, $R^2$ représente un radical alkylène à chaîne droite ou ramifiée contenant de 1 à 5 atomes de carbone et le plus souvent le radical isopropylidène et chacun des groupes X représente indépendamment l'hydrogène ou un halogène, avec une solution aqueuse d'un hydroxyde de métal alcalin pendant un temps suffisant pour transformer ce composé en son di-sel de métal alcalin solide.

(B) la séparation du sel ou de son hydrate solide du système aqueux;

(C) le mélange du sel ou de son hydrate solide avec un liquide organique présentant un point d'ébullition supérieur à celui de l'eau ou qui forme un azéotrope avec l'eau pour produire un système permettant l'élimination de l'eau, y compris de l'eau d'hydratation, du sel ou hydrate solide par évaporation et

(D) l'évaporation de l'eau du système.

2. Procédé selon la revendication 1, dans lequel le métal alcalin est le sodium.

3. Procédé selon la revendication 1, dans lequel on met en oeuvre l'étape A dans une bouillie aqueuse à une température inférieure à environ 45°C.

4. Procédé selon la revendication 3, dans lequel le liquide organique utilisé dans l'étape C est un liquide dans lequel l'eau et le di-sel de sodium anhydre sont pratiquement insolubles.

5. Procédé selon la revendication 4, dans lequel le liquide organique bout au-dessus de 105°C.

6. Procédé selon la revendication 5, dans lequel le liquide organique est le toluène ou l'o-dichlorobenzène.

7. Procédé selon la revendication 6, dans lequel on utilise dans l'étape A un excès d'hydroxyde de sodium pouvant aller jusqu'à environ 20%.

8. Procédé selon la revendication 7, dans lequel le liquide organique est le toluène.

9. Procédé selon la revendication 7, dans lequel le liquide organique est l'o-dichlorobenzène.

10. Procédé selon l'une des revendications 1 à 9, dans lequel le composé aromatique dihydroxylé est le bisphénol-A.

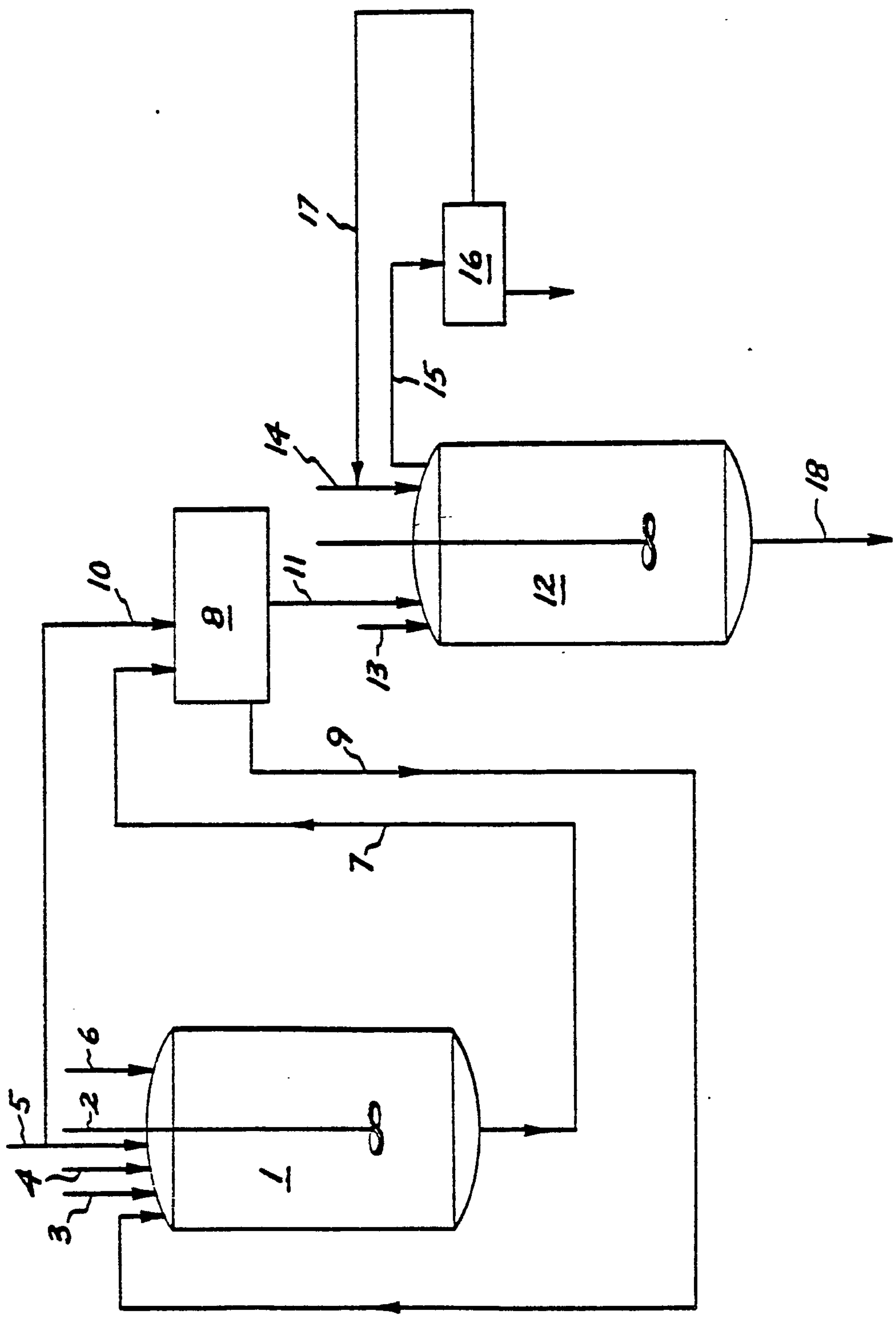
1
2
3
4
5
6
7
8
9
10
11
12
13
14
15
16
17
18